# EUROPÄISCHE PATENTANMELDUNG

(11) **EP 2 319 530 A1**
(43) Veröffentlichungstag der Anmeldung: **11.05.2011**
(21) Anmeldenummer: 10014636.4
(22) Anmeldetag: 25.01.2007
(51) Int. Cl.: A61K 38/18

(54) **Verfahren zur Behandlung und/oder Prophylaxe von Multipler Sklerose, sowie Verwendung von Erythropoietin zur Herstellung eines Arzneimittels zur intermittierenden Behandlung und/oder intermittierenden Prophylaxe von Multipler Sklerose**

(30) Priorität: 27.01.2006 DE 102006004008
(62) Teilanmeldung aus: 07703030.2
(71) Anmelder: Ehrenreich, Hannelore, 37085 Göttingen (DE)
(72) Erfinder: Ehrenreich, Hannelore, 37085 Göttingen (DE); Nave, Klaus-Armin, 37073 Göttingen (DE)
(74) Vertreter: Pfenning, Meinig & Partner GbR

(57) **Zusammenfassung**

Die vorliegende Erfindung betrifft ein Verfahren zur Behandlung und/oder Prophylaxe von Multipler Sklerose sowie die Verwendung von Erythropoietin hierzu sowie zur Herstellung eines Arzneimittels zur intermittierenden Behandlung und/oder intermittierenden Prophylaxe von Multipler Sklerose.

## Beschreibung

Die vorliegende Erfindung betrifft ein Verfahren zur Behandlung und/oder Prophylaxe von Multipler Sklerose sowie die Verwendung von Erythropoietin hierzu sowie zur Herstellung eines Arzneimittels zur intermittierenden Behandlung und/oder intermittierenden Prophylaxe von Multipler Sklerose.

Mit etwa 80 bis 110 Fällen von Multipler Sklerose (MS) pro 100.000 Personen ist die Multiple Sklerose die häufigste chronische Erkrankung des zentralen Nervensystems. Ein Drittel der Patienten zeigt dabei einen primären oder sekundären progressiven Verlauf der Multiplen Sklerose, für die bis heute noch keine Therapie verfügbar ist. Insbesondere steht keine Therapie zur Verfügung, die eine Verbesserung des Krankheitsbildes erzielen würde.

Erythropoietin ist ein körpereigenes Glycoprotein mit einem Molekulargewicht von 34.000 D. Es ist ein wesentlicher Wachstumsfaktor für die Produktion von Erythrozyten und wurde bereits 1977 erstmals isoliert.

Erythropoietin ist seit vielen Jahren in häufigem, klinischem Gebrauch bei Patienten mit renaler Anämie, bei Nephrodialyse, zur Gewinnung größerer Mengen autologen Blutes vor geplanten Operationen und geriet auch als Dopingmittel in die Schlagzeilen der Presse.

Dabei erwies sich Erythropoietin als ausgesprochen gut verträglich. Als relevante Nebenwirkung sind insbesondere die oft therapeutisch erwünschte Stimulation der Hämatopoiese mit Polyglobulie sowohl eine selten zu beobachtende arterielle Hypertonie zu nennen. Beide Auswirkungen sind hauptsächlich nach chronischer Erythropoietingabe zu erwarten. Ihnen ist im Bedarfsfall relativ einfach durch medikamentöse Behandlung bzw. Aderlass abzuhelfen. Unverträglichkeitsreaktionen bzw. anaphylaktische Reaktionen gehören bei Erythropoietin zu den Raritäten. Die WO 00/61164 offenbart den Einsatz von EPO zum Schutz neuronaler Gewebe, insbesondere auch des zentralen Nervensystems. In dieser Druckschrift wird beiläufig erwähnt, dass mit EPO auch Multiple Sklerose behandelt werden könne.

Unter EPO bzw. Erythropoietin im Sinne der vorliegenden Erfindung wird so wie in der WO 00/61164 jegliches Erythropoietin, ob nativ oder rekombinant, vom Mensch oder einem anderen Säugetier, ob in nativer Sequenzabfolge oder auch nach Sequenzänderungen oder Sequenzverkürzungen, jegliche Art von Erythropoietinanalogon, Erythropoietinfragmenten oder auch an den Erythropoietinrezeptor bindende Stoffe bzw. Erythropoietinagonisten betrachtet. Die Definition für Erythropoietin bzw. EPO gemäß der WO 00/61164 wird in die vorliegende Anmeldung vollständig übernommen. Demnach wird unter EPO und unter Erythropoietin jede Substanz verstanden, die bei systemischer Applikation EPO-aktivierte Rezeptoren aktivieren kann, also auch jede Art von Varianten, Fragmenten oder Analoga. Weitere verwendbare EPO-Varianten sind beispielsweise in den folgenden Druckschriften veröffentlicht:

Leist et al., Science 2004, Vol. 305, S. 239-242, WO 86/03520, WO 85/02610, WO 90/11354, WO 91/06667, WO 91/09955, WO 93/09222, WO 94/12650, WO 95/31560, WO 95/05465.

Eine Übersicht über bekannte EPO-Varianten und -Analoga, die auch sämtlich bei der vorliegenden Erfindung eingesetzt werden können, sowie über deren bekannte Einsatzgebiete findet sich in Brines und Cerami, Nature Reviews, Neuroscience, Juni 2005, Band 6, Seiten 484-494.

Insbesondere ist es bei der vorliegenden Erfindung nicht relevant, ob die eingesetzten EPO-Analoga bzw. Erythropoietinfragmente eine hämatopoietische Wirkung aufweisen oder nicht. Dies wird später genauer erläutert.

Die WO 00/61164 offenbart zwar die Verwendung von EPO für die Behandlung von Multipler Sklerose, gibt jedoch keinerlei experimentelle Daten oder Behandlungsregime an.

Aufgabe der vorliegenden Erfindung ist es daher, die Verwendung von Erythropoietin zur Behandlung von Multipler Sklerose so weiterzubilden, dass eine merkliche Stabilisierung bzw. Verbesserung des Krankheitsbildes erzielt wird. Diese Aufgabe wird durch das Verfahren nach Anspruch 1 sowie die Verwendung nach den Ansprüchen 14 und 15 gelöst. Vorteilhafte Weiterbildungen des erfindungsgemäßen Verfahrens sowie der erfindungsgemäßen Verwendungen werden in den jeweiligen abhängigen Ansprüchen gegeben.

Erfindungsgemäß wird nun beim Verfahren zur Behandlung und/oder Prophylaxe von Multipler Sklerose eines Säugetieres, insbesondere eines Menschen, das Erythropoietin intermittierend appliziert. Dies bedeutet, dass mit der vorliegenden Erfindung eine Intervallbehandlung vorgeschlagen wird. Besonders vorteilhaft ist es dabei, wenn die Behandlung aus einer Abfolge von Zeiträumen unter Applikation von EPO (Applikationszeitraum) und Zeiträumen ohne Applikation von EPO (applikationsfreie Zeiträume) besteht.

Die einzelnen Zeiträume betragen dabei mehrere Wochen. Besonders vorteilhaft hat sich eine Abfolge herausgestellt, bei dem jeder Applikationszeitraum 12 bis 48 Wochen, vorteilhafterweise 18 bis 36 Wochen, vorteilhafterweise 24 bis 28 Wochen, dauert, während die applikationsfreien Zeiträume 8 bis 53 Wochen, vorteilhafterweise 16 bis 28 Wochen dauern. Innerhalb der Applikationszeiträume kann die Dosierung variiert werden, beispielsweise zuerst ein Zeitraum mit einer wöchentlichen Applikation und einem nachfolgenden Zeitraum mit einer zweiwöchentlichen Applikation.

Die vorliegende Erfindung dient insbesondere der Verwendung von Erythropoietin in einem derartigen Verfahren bzw. der Verwendung von Erythropoietin zur Herstellung eines Arzneimittels, mit dem das beschriebene Verfahren durchgeführt werden kann.

Die Dosierung liegt dabei jeweils bei den in den Ansprüchen beschriebenen Werten, besonders vorteilhaft in einem Dosisbereich von 5.000 IE bis 100.000 IE (Internationale Einheiten) pro Woche oder pro Gabe.

Unter den genannten Intervalldosierschemata kommt es überraschenderweise zu einer stetigen Verbesserung der klinischen Symptomatik während der Behandlung. Das verbesserte Niveau wird erstaunlicherweise im Intervall gehalten und der zweite Zyklus erzeugt eine weitere Verbesserung.

Überraschenderweise zeigte sich eine Verbesserung der Symptomatik nicht nur bei chronisch progredienter Multipler Sklerose, sondern auch bei der schubförmig verlaufenden Multiplen Sklerose. Insbesondere bei der chronisch progredienten Multiplen Sklerose wäre während des behandlungsfreien Intervalls eine Verschlechterung der Symptomatik zu erwarten gewesen. Es trat jedoch auch hier eine Stabilisierung ein.

Die erfindungsgemäße Intervallbehandlung ist ein innovativer Ansatz im gesamten Konzept der Neuroprotektion, die im Fall von EPO bei Multipler Sklerose obendrein die Tatsache ausnutzt, dass es durch die halbjährliche Erythropoietinbehandlung zu einem latenten, gewünschten Eisenmangel kommt. Da Eisenmangel zusätzlich zur bekannten neuroprotektiven EPO-Wirkung bei chronisch entzündlichen Erkrankungen wie der Multiplen Sklerosen vorteilhaft sein kann, wird vorteilhafterweise weder in der Behandlungs- noch in der behandlungsfreien Phase Eisen substituiert. Die behandlungsfreie Phase dient daher auch der langsamen, durch ausgewogene Ernährung zustande kommenden Wiederfüllung der geleerten Eisenspeicher.

Insbesondere im Falle der Verwendung von EPO mit hämatopoietischer Wirkung wird folglich die neuroprotektive Wirkung ergänzt durch den durch die EPO-Behandlung erzeugten latenten Eisenmangel.

Die erfindungsgemäße Wirkung wird jedoch auch bereits unter Verwendung von EPO-Derivaten bzw. -Varianten ohne hämatopoietische Wirkung erzielt.

Die vorstehende Erläuterung bezieht sich auf das Verfahren, die Erfindung ist jedoch nicht nur auf das therapeutische Verfahren gerichtet, sondern auch auf die Verwendung von EPO in einem derartigen Verfahren als auch die Verwendung von EPO zur Herstellung eines Arzneimittels zur Verwendung in einem derartigen Verfahren.

Im folgenden werden einige Beispiele eines erfindungsgemäßen Einsatzes von Erythropoietin-α (Handelsname Erypo/Eprex) bzw. Erythropoietin-β (Handelsname Neorecormon) gegeben.

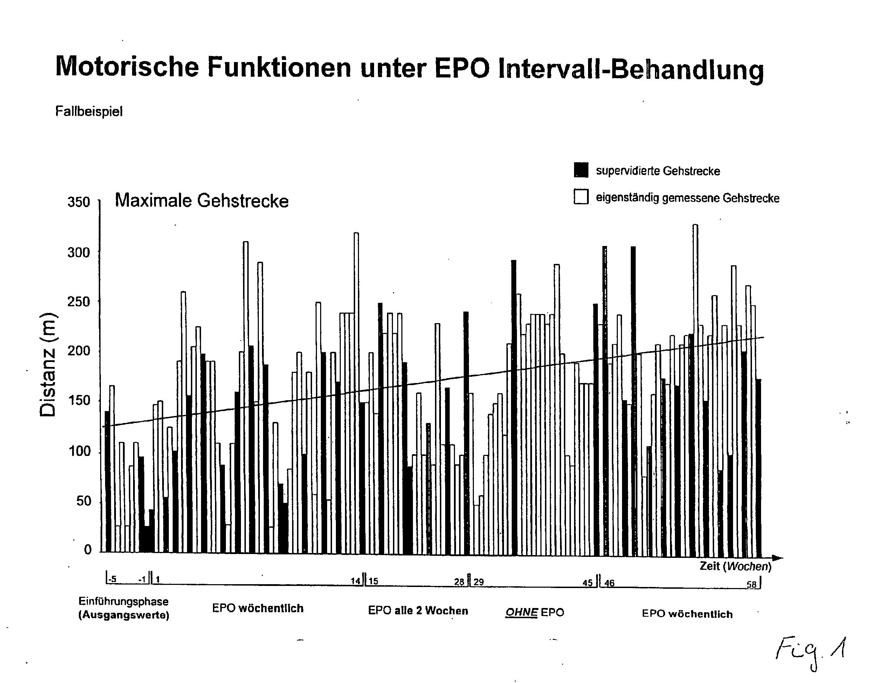

Figur 1 zeigt den Verlauf der maximalen Gehstrecke (Prototyp motorischer Funktionsmessung bei Multipler Sklerose) bei einer Patientin (Fallbeispiel), welche über einen Zeitraum von 58 Wochen eine EPO-Intervallbehandlung hatte. Wie aus der Figur ersichtlich, hatte die Patientin eine Einführungsphase von insgesamt fünf Wochen zur Bestimmung der Baseline-Werte ihrer Gehstrecke. Darauf folgte eine wöchentliche Gabe von EPO über 14 Wochen. Ab der 15. Woche bis einschließlich 28. Woche erhielt sie EPO alle zwei Wochen. Von Woche 29 bis 45 wurde eine Behandlungsphase gemacht, d.h. die Patientin erhielt kein EPO. Von Woche 46 bis 58 wurde sie wiederum wöchentlich mit EPO behandelt. Die eingezeichnete "Trendlinie" zeigt eine über den Gesamtverlauf der Untersuchung stetige Zunahme der maximalen Gehstrecke. Dieser Verlauf ist bei chronisch progredienter MS in gegenläufiger Richtung (progrediente Verschlechterung der Gehstrecke) zu erwarten.

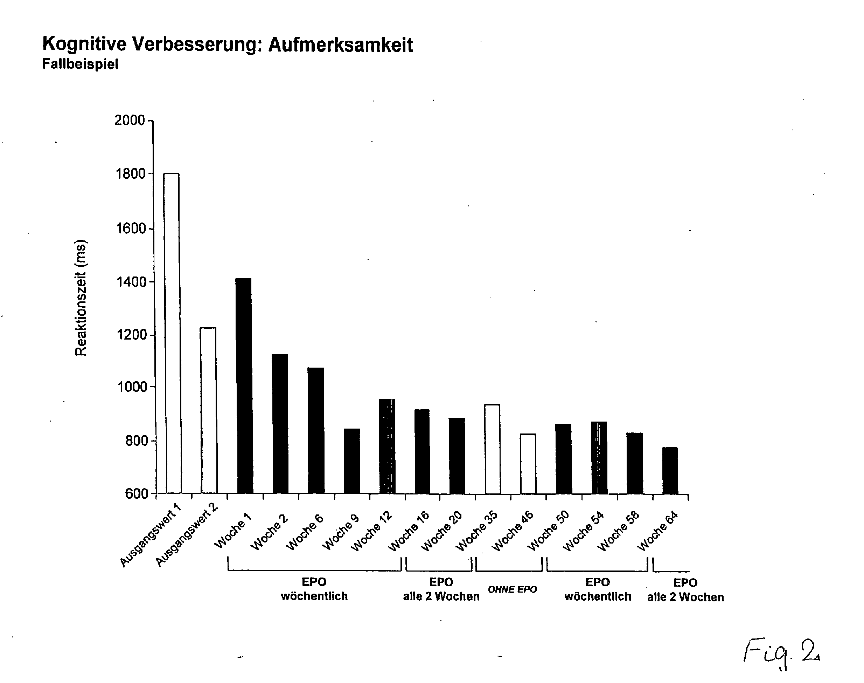

Figur 2 zeigt bei derselben Patientin wie in Fig. 1 (EPO-Intervallbehandlung) eine Verbesserung der Aufmerksamkeitsleistung über die unterschiedlichen Behandlungsphasen hinweg, ohne dass Einbrüche in der Leistung im therapiefreien Intervall zu beobachten wären.

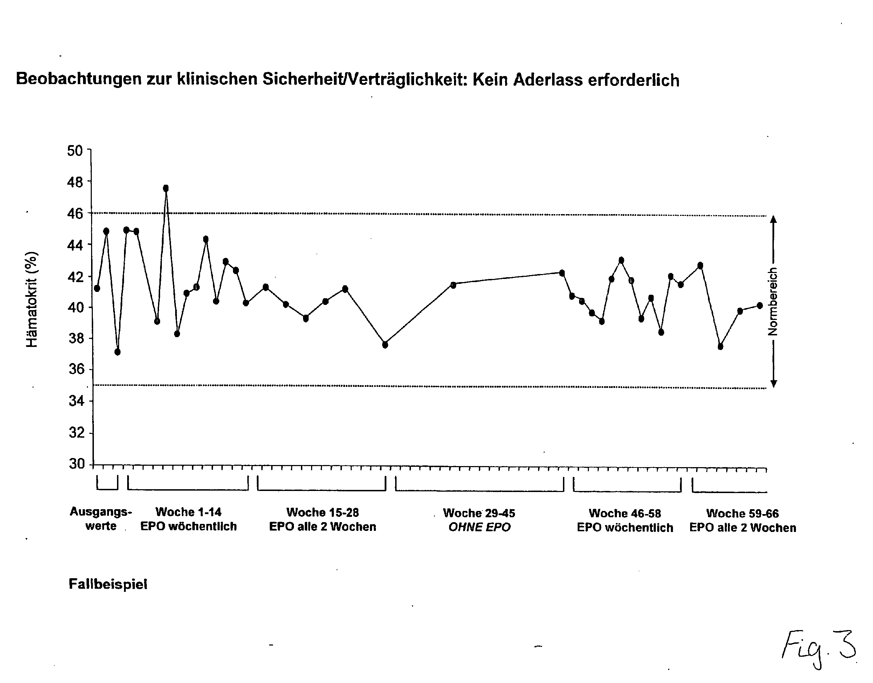

Figur 3 demonstriert den Verlauf des Hämatokritwertes bei derselben Patientin wie in Fig. 1 über den gesamten Untersuchungszeitraum. Es ergibt sich, dass in diesem Fallbeispiel kein Wert außerhalb des Normbereiches fällt. Es war bei dieser Patientin über die gesamte Behandlungsdauer hinweg kein Aderlass erforderlich.

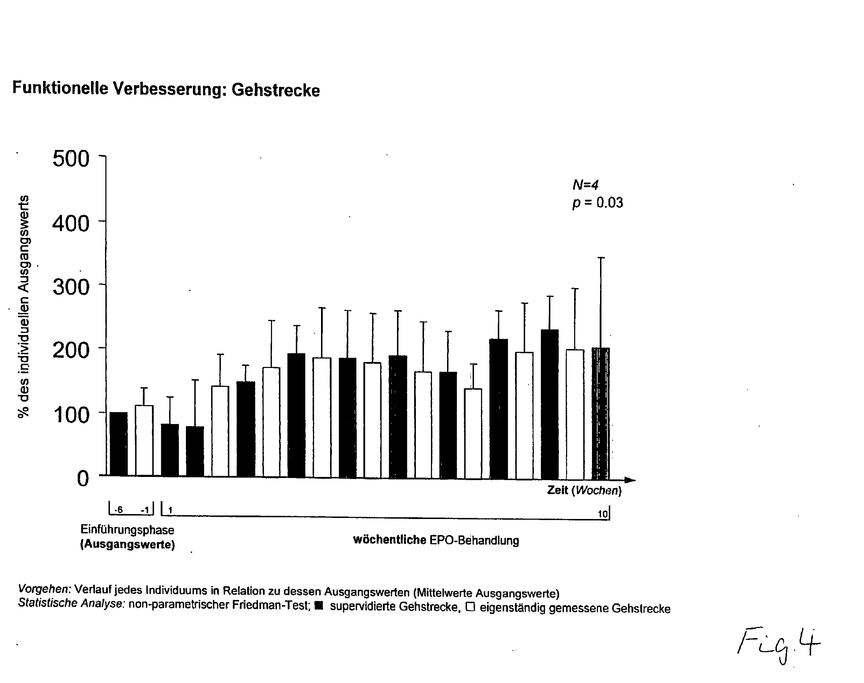

Figur 4 fasst die funktionelle Verbesserung der Gehstrecke bei vier Patienten zusammen, welche in gleicher Weise wie die in Fig. 1 dargestellte Patientin behandelt wurden. Es zeigt sich bereits über zehn Wochen EPO-Behandlung eine signifikante Verbesserung.

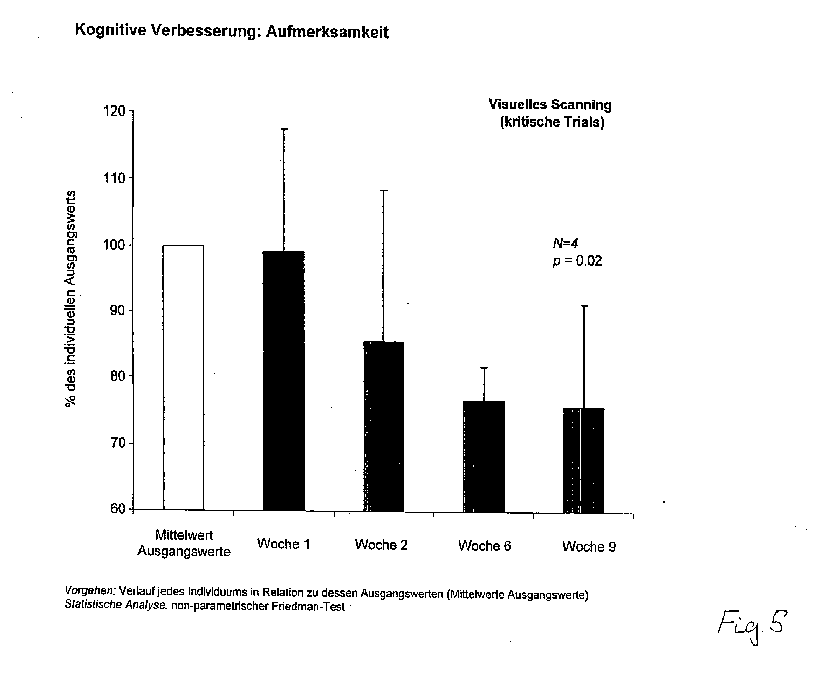

Figur 5 zeigt eine Zusammenfassung der Aufmerksamkeitsleistung (visuelles Scanning) bei den in Figur 4 gezeigten vier Patienten über einen Zeitraum von insgesamt neun Wochen. Die Verbesserung der kognitiven Leistung ist signifikant.

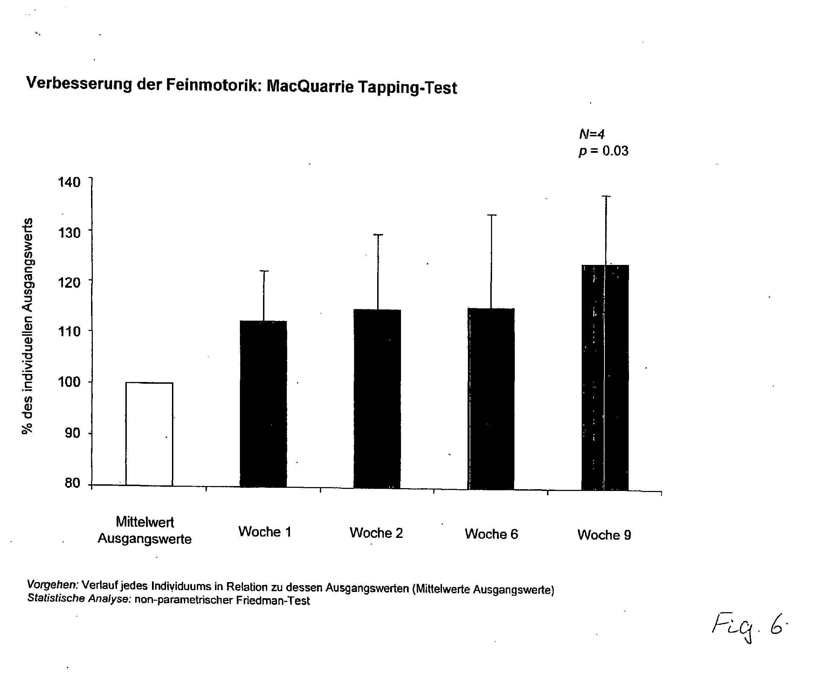

Figur 6 zeigt ebenfalls in der Gruppe der vier Patienten, die in Fig. 4 dargestellt wurden, eine signifikante Verbesserung der Feinmotorik über die ersten neun Wochen der wöchentlichen EPO-Behandlung hinweg.

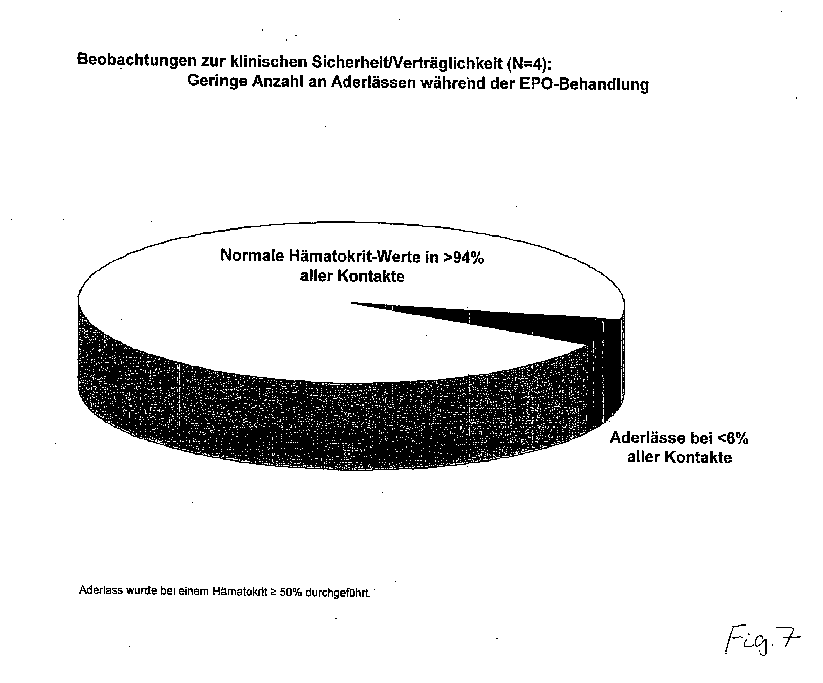

Figur 7 demonstriert unter Verwendung des in Fig. 4 dargestellten Patientenkollektivs, dass bei sehr guter klinischer Sicherheit/Verträglichkeit auch des klassischen (hämatopoietischen) EPO die Notwendigkeit der Durchführung von Aderlässen bei MS-Patienten sich auf eine geringe Zahl beschränkt.

Dabei wurden insgesamt vier Patienten mit Erythropoietin-α bzw. Erythropoietin-β (Neorecormon) behandelt.

Zusammenfassend lässt sich feststellen, dass sich erwiesen hat, dass eine derartige Langzeitbehandlung mit EPO von den Patienten gut vertragen wird.

Insbesondere zeigte sich, dass das angewandte Therapieregime sowohl die motorische, kognitive als auch die neurophysiologische Leistung verbesserte.

## Patentansprüche

1. Verwendung von Erythropoietin zur Herstellung eines Arzneimittels zur intermittierenden Behandlung und/oder intermittierenden Prophylaxe von Multipler Sklerose.

2. Erythropoietin zur intermittierenden Behandlung und/oder intermittierenden Prophylaxe von Multipler Sklerose.

3. Erythropoietin oder Verwendung von Erythropoietin nach einem der vorhergehenden Ansprüche, **dadurch gekennzeichnet, dass** zur intermittierenden Behandlung oder Prophylaxe das EPO in Intervallen appliziert wird, die von Zeiträumen unterbrochen werden, in denen kein EPO appliziert wird.

4. Erythropoietin oder Verwendung von Erythropoietin nach einem der vorhergehenden Ansprüche, **dadurch gekennzeichnet, dass** die Applikation des EPO intermittierend in mindestens zwei Applikationszeiträumen mit einer Dauer von jeweils mindestens zwei Wochen erfolgt, wobei zwischen zwei Applikationszeiträumen applikationsfreie Zeiträume mit einer Dauer von mindestens zwei Wochen vorgesehen sind, in denen kein EPO appliziert wird.

5. Erythropoietin oder Verwendung von Erythropoietin nach einem der vorhergehenden Ansprüche, **dadurch gekennzeichnet, dass** die Applikation parenteral/systemisch erfolgt.

6. Erythropoietin oder Verwendung von Erythropoietin nach einem der vorhergehenden Ansprüche, **dadurch gekennzeichnet, dass** die Applikation vaskulär, intranasal und/oder per Inhalation erfolgt.

7. Erythropoietin oder Verwendung von Erythropoietin nach einem der vorhergehenden Ansprüche, **dadurch gekennzeichnet, dass** die Applikation intravenös, subcutan und/oder intramuskulär erfolgt.

8. Erythropoietin oder Verwendung von Erythropoietin nach einem der vorhergehenden Ansprüche, **dadurch gekennzeichnet, dass** das Säugetier ein Mensch ist.

9. Erythropoietin oder Verwendung von Erythropoietin nach einem der vorhergehenden Ansprüche, **dadurch gekennzeichnet, dass** als EPO natives oder rekombinantes Erythropoietin, ein Erythropoietin-Analogon, ein Erythropoietin-Fragment, ein an den Erythropoietin-Rezeptor bindender Stoff, ein Erythropoietin-Agonist oder dergleichen verwendet wird.

10. Erythropoietin oder Verwendung von Erythropoietin nach einem der vorhergehenden Ansprüche, **dadurch gekennzeichnet, dass** EPO in nativer Form, synthetischer und/oder rekombinanter Form mit oder ohne Sequenzänderungen oder Sequenzvarianten verwendet wird.

11. Erythropoietin oder Verwendung von Erythropoietin nach einem der vorhergehenden Ansprüche, **dadurch gekennzeichnet, dass** als EPO ein EPO mit hematopoietischer Wirkung und/oder ohne hematopoietische Wirkung verwendet wird.

12. Erythropoietin oder Verwendung von Erythropoietin nach einem der vorhergehenden Ansprüche, **dadurch gekennzeichnet, dass** das EPO in einer Dosis von 1000 IE bis 200000 IE, vorteilhafterweise von 5000 IE bis 100000 IE pro Woche, pro Applikation und/oder pro Applikationszeitraum gegeben wird.

13. Erythropoietin oder Verwendung von Erythropoietin nach einem der vorhergehenden Ansprüche, **dadurch gekennzeichnet, dass** ein Applikationszeitraum 12 bis 48 Wochen, vorteilhafterweise 18 bis 36 Wochen, vorteilhafterweise 24 bis 28 Wochen dauert.

14. Erythropoietin oder Verwendung von Erythropoietin nach einem der vorhergehenden Ansprüche, **dadurch gekennzeichnet, dass** ein applikationsfreier Zeitraum 8 bis 53 Wochen, vorteilhafterweise 16 bis 28 Wochen dauert.

15. Erythropoietin oder Verwendung von Erythropoietin nach einem der vorhergehenden Ansprüche, **dadurch gekennzeichnet, dass** mindestens einer der Applikationszeiträume aus zwei Teilzeiträumen besteht, wobei in einem ersten Teilzeitraum EPO wöchentlich appliziert wird und in einem nachfolgenden zweiten Teilzeitraum EPO zweiwöchentlich appliziert wird.
